# EUROPÄISCHE PATENTSCHRIFT

(11) **EP 0 439 694 B1**
(45) Veröffentlichungstag und Bekanntmachung des Hinweises auf die Patenterteilung: **11.01.1995**
(21) Anmeldenummer: 90120991.6
(22) Anmeldetag: 02.11.1990
(51) Int. Cl.: G01N 33/26

(54) **Verfahren und Vorrichtung zur Messung des Alkoholgehalts der Feuchtflüssigkeit eines Feuchtwerks einer Offset-Druckmaschine**
Method and device for measuring the alcohol content of a damping liquid in a damping device of an offset printing machine
Procédé et dispositif pour mesurer le taux d'alcool du liquide de mouillage à l'alcool d'une machine d'imprimerie offset

(30) Priorität: 27.01.1990 DE 4002401
(43) Veröffentlichungstag der Anmeldung: 07.08.1991
(73) Patentinhaber: MAN Roland Druckmaschinen AG, 63012 Offenbach/Main (DE)
(72) Erfinder: Rodriguez-Giles, Jorge M., Dr., W-4800 Bielefeld (DE)

(56) Entgegenhaltungen:
- EP-A- 0 264 554
- DE-A- 3 309 458
- PATENT ABSTRACTS OF JAPAN, Band 11, Nr. 340 (P-634)[2787], 07 November 1987#
- PATENT ABSTRACTS OF JAPAN, Band 7, Nr. 38 (P-176)[1183], 16 Februar 1983#

## Beschreibung

Die Erfindung betrifft ein verfahren zur Messung des Alkoholgehalts der Feuchtflüssigkeit eines Feuchtwerks einer Offset-Druckmaschine, wobei aus der Feuchtflüssigkeit durch Durchperlen von Luft eine Gasmischung erzeugt wird, die Luft, Wasserdampf und brennbaren Alkoholdampf enthält, worauf in einem Meßgerät aus dem Alkoholdampf der Gehalt an Alkohol ermittelt wird. Außerdem bezieht sich die Erfindung auf eine Vorrichtung zur Durchführung dieses Verfahrens mit einem in ein Feuchtmittelgefäß eingetauchten, glockenförmigen Behälter, in den ein in das Feuchtmittel eintauchendes Rohr für die Zuführung von Druckluft eingesetzt ist und an den ein Meßrohr angeschlossen ist, das das Meßgas dem Meßgerät zuleitet.

Ein Verfahren und eine Vorrichtung mit diesen Merkmalen ist in der deutschen Patentanmeldung P 38 28 325.5-52 bzw. in der europäischen Patentanmeldung 89 115 091.4 beschrieben. Diese älteren Patentanmeldungen sind allerdings für die benannten Länder Italien, Österreich und Spanien kein Stand der Technik, so daß für diese Länder die Aufstellung eines gesonderten Anspruchssatzes vorbehalten bleibt.

Dieses Verfahren und die Vorrichtung haben sich an und für sich bewährt. Sie beruhen darauf, daß der Anteil an Alkohol, der aus der mit Alkohol versetzten Feuchtflüssigkeit beim Durchperlen der Luft mitgerissen wird, proportional zum Anteil des Alkohols in der Feuchtflüssigkeit ist. Der in dem Gasgemisch vorliegende Alkoholanteil kann dann sehr einfach, genau und zuverlässig mit Hilfe eines erprobten Meßgeräts bestimmt werden.

Die meisten der hier zum Einsatz kommenden Meßgeräte haben allerdings die Eigenschaft, daß sich ihr Nullpunkt von Zeit zu Zeit verschiebt. Dies wurde insbesondere bei Wärmetönungsfühlern festgestellt, die in ihrem grundsätzlich Aufbau bekanntlich eine Wheatstonesche Brücke beinhalten, bei der zwei Widerstände aus dünnen Platindrähten gebildet sind. An einem der Platindrähte, der vom Meßgas umspült wird, verbrennt dabei durch eine katalytische Reaktion der gasförmige Alkohol. Die hierdurch hervorgerufene Temperaturerhöhung wird in der Wheatstoneschen Brücke ausgewertet und in einer angeschlossenen Auswerteschaltung zur Anzeige gebracht. Bei derartigen Meßgeräten wurde, wie erwähnt, festgestellt, daß sich von Zeit zu Zeit der Nullpunkt verschiebt. Dies beruht offenbar auf einer Alterung der Sensoren des Meßgeräts. Entspricht beispielsweise bei der Ingebrauchnahme des Geräts einem Anteil von Null Prozent Alkohol eine Spannung von Null Volt und einem Anteil zehn Prozent Alkohol eine Spannung von fünf Volt des Meßgeräts, so entsprechen nach einer gewissen Betriebszeit den erwähnten Alkoholanteilen beispielsweise Spannungen von eins bzw. sechs Volt.

Der Erfindung liegt daher die Aufgabe zugrunde, ein Verfahren und eine Vorrichtung vorzuschlagen, womit auf einfache Weise und zuverlässig die Nullpunktverschiebung des Meßgeräts ausgeglichen werden kann.

Zur Lösung dieser Aufgabe ist das erfindungsgemäße Verfahren dadurch gekennzeichnet, daß von Zeit zu Zeit Luft ohne Anteile an brennbarem Alkoholdampf durch das Meßgerät hindurchgeleitet wird und daß die Ergebnisse dieser Kontrollmessungen für einen Nullabgleich des Meßgeräts verwendet werden.

Die Kontrollmessungen wird man in der Regel von einem geeigneten Zeitschalter gesteuert periodisch durchführen, und zwar immer dann, wenn zu erwarten ist, daß sich eine Nullpunktverschiebung ergeben hat. Die Kontrollmessungen können aber auch jeweils von Hand eingeleitet werden. Die Ergebnisse der Kontrollmessungen werden vorzugsweise in der elektronischen Schaltung so ausgewertet, daß dadurch automatisch, ggf. auch nach Betätigung eines gesonderten Schalters, die Nullpunktkorrektur des Meßgeräts durchgeführt wird.

Das Prinzip dieses Nullpunkt-Korrekturverfahrens beruht also darauf, von Zeit zu Zeit Luft ohne Belastung mit brennbaren Dämpfen durch den Meßfühler bzw. das Meßgerät hindurchzuleiten. Zeigt das Meßgerät bei diesen Kontrollmessungen einen Meßwert an, so muß dieser durch den Nullabgleich zu Null gebracht werden.

Zur Lösung der erwähnten Aufgabe ist die erfindungsgemäße Vorrichtung dadurch gekennzeichnet, daß das Druckluftrohr über eine Zweigleitung mit steuerbarem Ventil direkt mit dem Meßgerät verbunden ist. Man macht sich hier zunutze, daß Druckluft ohnedies vorhanden ist, die ja die Feuchtflüssigkeit durchperlt und diese Druckluft wird direkt, d.h. ohne den Umweg über das Feuchtwerk, dem Meßfühler zugeleitet.

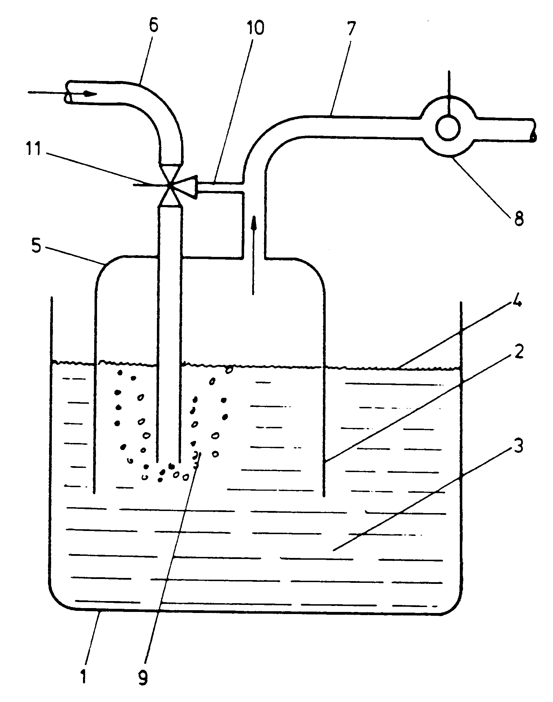

Die Erfindung wird im folgenden anhand eines Ausführungsbeispieles näher erläutert, aus dem sich weitere wichtige Merkmale ergeben. Die Figur zeigt schematisch den grundsätzlichen Aufbau einer erfindungsgemäßen Vorrichtung.

Ein - zentrales oder dezentrales - Feuchtmittelgefäß 1 einer Offset-Druckmaschine ist mit Feuchtflüssigkeit 3 gefüllt. In die Feuchtflüssigkeit taucht ein an der Unterseite offener Behälter 2 ein. Der Flüssigkeitsspiegel der Feuchtflüssigkeit 3 ist bei Pos. 4 angedeutet.

In die obere Abdeckung 5 des Behälters 2 ist ein Druckluftrohr 6 eingesetzt, das in die Feuchtflüssigkeit 3 eintaucht. In das Rohr wird in Richtung des Pfeiles Druckluft eingeblasen und zeichnerisch ist auch dargestellt, daß die Luftbläschen 9 nach oben zum Flüssigkeitsspiegel 4 perlen. Auf diesem Wege haben sie sich mit Alkohol angereichert und das sich dann ergebende Gasgemisch aus Luft, Wasserdampf und brennbarem Alkoholdampf verläßt den Innenraum des glockenförmigen Behälters 2 in Richtung des Pfeiles über ein Meßrohr 7. Es gelangt dann durch ein Meßgerät 8, wo der Anteil an brennbarem Alkoholdampf in dem Gasgemisch gemessen wird.

Die bisher beschriebene Vorrichtung ist schon in der erwähnten älteren Patentanmeldung P 38 28 325.5-52 offenbart. (Vgl. die DE 38 28 325-A1 oder die EP 335 688-A2; das dort offfenbarte Meßprinzip wird grundsätzlich auch bei dieser Erfindung angewendet.) Es sei außerdem darauf hingewiesen, daß bei Feuchtwerken von Offset-Druckmaschinen der Alkoholgehalt der Feuchtflüssigkeit auf einen vorbestimmten Wert konstant gehalten werden soll und zu diesem Zweck muß der Alkoholgehalt gemessen werden. Durch dosierte Zugabe von Alkohol erhält man die gewünschte Konstanthaltung des Alkoholgehalts der Feuchtflüssigkeit. Auch dies ist schon Gegenstand der erwähnten älteren Patentanmeldungen.

Erfindungsgemäß sind die Rohre 6, 7 über eine Zweigleitung 10 mit einem steuerbaren Ventil 11 miteinander verbunden. Das Ventil ist beispielsweise als Dreiwegeventil ausgebildet, so daß die Druckluft entweder in das Gefäß 1 oder über die Zweigleitung 10 direkt in das Meßgerät 8 gelangt, ggf. über einen Teil des Meßrohres 7. Das Ventil 11 kann ggf. auch in der Zweigleitung 10 angeordnet sein und die Zweigleitung dann öffnen oder schließen, nämlich wenn man davon ausgehen kann, daß der durch den Flüssigkeitsspiegel im Gefäß 1 bewirkte Gegendruck ausreicht, keine Luft mehr durch die Flüssigkeit mehr hindurchperlen zu lassen.

Über eine geeignete Steuerung betätigt man von Zeit zu Zeit das Ventil 11 derart, daß reine Luft, d.h. ohne den Umweg über die Flüssigkeit 3, direkt dem Meßgerät 8 zugeleitet wird. Dadurch mißt man den Nullpunkt des Meßgeräts. Hat sich dieser verschoben, so kann man den Nullpunkt sofort abgleichen.

## Patentansprüche

1. Verfahren zur Messung des Alkoholgehalts der Feuchtflüssigkeit eines Feuchtwerks einer Offset-Druckmaschine, wobei aus der Feuchtflüssigkeit durch Durchperlen von Luft eine Gasmischung erzeugt wird, die Luft, Wasserdampf und brennbaren Alkoholdampf enthält, worauf in einem Meßgerät aus dem Alkoholdampf der Gehalt an Alkohol ermittelt wird,
**dadurch gekennzeichnet**,
daß von Zeit zu Zeit Luft ohne Anteile an brennbarem Alkoholdampf durch das Meßgerät hindurch geleitet wird und daß die Ergebnisse dieser Kontrollmessungen für einen Nullabgleich des Meßgeräts verwendet werden.

2. Vorrichtung zur Durchführung des Verfahrens nach Anspruch 1 mit einem in ein Feuchtmittelgefäß eingetauchten, glockenförmigen Behälter (5), in den ein in das Feuchtmittel eintauchendes Rohr (6) für die Zufuhr von Druckluft eingesetzt ist und an den ein Meßrohr (7) angeschlossen ist, das das Meßgas dem Meßgerät (8) zuleitet,
**dadurch gekennzeichnet**,
daß das Druckluftrohr (6) über eine Zweigleitung (10) mit steuerbarem Ventil (11) direkt mit dem Meßgerät (8) verbunden ist.

## Claims

1. Process for measuring the alcohol content of the damping liquid of a damping unit of an offset printing press wherein from the damping liquid a gas mixture is generated by bubbling air through it which contains air, water vapour and combustible alcohol vapour, whereon the content of alcohol is determined from the alcohol vapour in a measuring device, characterised in that from time to time air without a proportion of combustible alcohol vapour is fed through the measuring device and that the results of these control measurements are used for zero compensation of the measuring device.

2. Device for carrying out the process according to Claim 1 with a bell-shaped container (5) dipping into a damping agent vessel in which is set a tube (6) dipping into the damping agent for the feed of compressed air and to which a measuring tube (7) is connected which feeds the measuring gas to the measuring device (8), characterised in that the compressed air tube (6) is connected via a branch lead (10) with a controllable valve (11) directly with the measuring device (8).

## Revendications

1. Procédé pour mesurer la teneur en alcool du liquide de mouillage d'une unité de mouillage d'une machine d'impression offset, un mélange gazeux étant créé à partir du liquide de mouillage par barbotage d'air, ledit mélange contenant de l'air, de la vapeur d'eau et de la vapeur d'alcool combustible, la teneur en alcool étant déterminée dans un appareil de mesure à partir de la vapeur d'alcool, caractérisé en ce que, de temps en temps, de l'air, sans portion de vapeur d'alcool combustible, est amené à travers l'appareil de mesure, et en ce que les résultats de ces mesures de contrôle sont utilisés pour une compensation à zéro de l'appareil de mesure.

2. Dispositif pour la mise en oeuvre du procédé selon la revendication 1, comportant un récipient (5), en forme de cloche, plongé dans un récipient d'agent de mouillage, dans lequel est placé un tube (6) plongeant dans l'agent de mouillage pour l'amenée d'air comprimé et auquel est raccordé un tube de mesure (7), qui amène le gaz de mesure à l'appareil de mesure (8),
caractérisé en ce que le tube d'air comprimé (6) est relié directement à l'appareil de mesure (8) par un conduit de dérivation (10) à soupape commandable (11).
